# EUROPEAN PATENT SPECIFICATION

(11) **EP 2 639 582 B1**
(45) Date of publication and mention of the grant of the patent: **01.07.2015**
(21) Application number: 12001781.9
(22) Date of filing: 15.03.2012
(51) Int. Cl.: G01N 33/00

(54) **Odour and/or gas identification system**
Geruchs- und/oder Gaserkennungssystem
Système d'identification d'odeur et/ou gaz

(43) Date of publication of application: 18.09.2013
(73) Proprietor: Sensirion AG, 8712 Stäfa (CH)
(72) Inventor: Mayer, Felix, CH-8712, Stäfa, (CH); Graf, Markus, CH-8005, Zürich, (CH)
(74) Representative: Toleti, Martin

(56) References cited:
- US-A1- 2003 172 717
- AMY LOUTFI ET AL: "Odor Recognition for Intelligent Systems", IEEE INTELLIGENT SYSTEMS, IEEE, US, vol. 23, no. 1, 1 January 2008 (2008-01-01), pages 41-48, XP011200698, ISSN: 1541-1672
- ANAND D MANE ET AL: "Explosive detection with mobile telephony an attempt towards a safe ambience", SIGNAL PROCESSING, COMMUNICATION, COMPUTING AND NETWORKING TECHNOLOGIES (ICSCCN), 2011 INTERNATIONAL CONFERENCE ON, IEEE, 21 July 2011 (2011-07-21), pages 187-191, XP031940738, DOI: 10.1109/ICSCCN.2011.6024541 ISBN: 978-1-61284-654-5

## Description

### Technical Field

The present invention relates to an odour and/or gas identification system and to a method for identifying an odour and/or a gas.

### Background Art

In today's world of pervasive electronic devices, it would be desirable to get support in identifying an odour and/or gas.

In US 2003/0172717 A1, an odour measuring apparatus is disclosed capable of determining the similarity of the odour of an unknown sample with high objectivity and performing measurements with sensitivity similar to that of the human nose. An m-dimensional space is created from detection signals of m pieces of odour sensors, and a standard odour vector representing the result of measurement of a standard odour and another vector representing the result of measurement of an unknown sample are drawn in the space. From the angle theta between the two vectors, a degree of similarity is determined within the range from 0 to 100%, where the degree of similarity is corrected taking account of the difference in sensitivity between the odour sensor and the human nose. The degree of similarity of the unknown odour is calculated for each of plural standard odours measured beforehand, and which standard odour is the closest to the odour of the unknown sample is objectively shown by numeral values.

"Odor Recognition for intelligent Systems" by A. Loutfi and S. Coradeschi, IEEE Intelligent Systems, January/February 2008, p. 41 - 48, refers to a robot movable on the floor. The robot includes an electronic nose for sensing odours.

### Disclosure of the Invention

This problem is solved by odour and/or gas identification systems according to the features of claims 1 and 2, and by methods for identifying an odour and/or gas according to the features of claims 11 and 12.

The odour and/or gas identification system comprises a portable electronic device, a database, and an evaluation unit.

The portable electronic device comprises a chemical sensor which is sensitive to different chemical analytes. By means of such chemical sensor a gas in the environment of the portable electronic device may be investigated at least as to the absence or presence of the subject analytes the chemical sensor is sensitive to. The detection of chemical substances or compounds contained in such gas may be of interest to a user. The gas may contain substances or compounds that can be smelled by a human being and in such case the gas composition may be perceived as an odour, scent or flavour, and such gas would be denoted and understood by the human being by an identifier for the odour. Other gases may not contain substances that can be smelled by a human being or may contain substances that can be smelled, however, only at a very little degree such that a concentration is not sufficient for a human to smell. Still such gas may be denoted und understood by a human being by an identifier denoting a corresponding chemical formula or a corresponding name.

Analytes may include chemical substances and/or chemical compounds, and may specifically include one or more of, for example, CO2, NOX, ethanol, CO, ozone, ammonia, formaldehyde, or xylene without limitation. The chemical sensor is adapted to detect at least one property of at least two different analytes, and preferably is sensitive to five or more different analytes. Hence, the gas supplied to the chemical sensor may be analyzed by means of the chemical sensor as to if and which of the chemical substances or compounds the chemical sensor is sensitive to are present in the gas supplied. A combination of analytes detected in the gas supplied may suggest for a certain odour or for a certain gas. It is always subject to a design of the chemical sensor as to how many different analytes > 1 and/or how many different properties of an analyte the chemical sensor is sensitive to.

The chemical sensor is capable of measuring one or more properties of multiple different analytes. Hence, the chemical sensor may be understood as a sensor device for detecting one or even more properties of more than one analyte. A property may, for example, be a concentration of an analyte in a gas which, for example, may be the air surrounding the device. Other properties may be, for example, chemical properties such as a binding energy of an analyte. Or, the chemical sensor may comprise at least sensor material, e.g. in form of a layer, an analyte may interact with. As a result, an electrical property of the sensor material may be modified upon interaction such as its electrical conductance, which principle preferably is applied in metal oxide chemical sensors, or an optical property of the sensor material may be modified such as its transmission rate, for example. Then, the electrical or optical property of a combination of the analyte and the sensor material is measured and allows a conclusion as to the analyte, such as by way of comparison to a property of the sensor material measured without the presence of the analyte. It is noted that for the different analytes the chemical sensor is sensitive to it is not required to always measure the same property per analyte. Different properties may be measured for different analytes.

Specifically, the chemical sensor may be embodied as a sensor array. A sensor array may comprise a set of sensor cells, wherein each sensor cell may provide a layer of a material exhibiting different sensitivity such that each cell of the sensor array may specifically be mainly sensitive to a different analyte and as such may enable the portable electronic device to detect the presence or absence or concentration of such analyte. "Mainly" in this context shall mean that a sensor cell is more sensitive to the subject analyte than to other analytes. In other variants, each sensor cell may provide a sensor material, e.g. in form of a layer and also denoted as sensitive layer, an analyte may interact with. As a result of the interaction, which e.g. may be a catalytic reaction, an electrical property of the sensor material may be modified, such as its electrical conductance, which principle preferably is applied in metal oxide chemical sensors, or an optical property may be modified such as its transmission rate, for example. However, a sensor cell of such sensor array may in one embodiment exhibit not only sensitivity to its main analyte, but also to analytes other than the main analyte since such sensor cell may exhibit a cross-sensitivity to one or more analytes possibly representing main analytes for other cells. In another embodiment, the chemical sensor may be a single sensor cell, e.g. with a single layer, which however, may be sensitive to multiple different analytes. Such single cell may, in one embodiment, be sensitive to different analytes only under different operating conditions. For example, the sensor cell may mainly be sensitive to a first analyte x when being heated to a first temperature tx, and may mainly be sensitive to a second analyte y when being heated to a second temperature ty which is different from the first temperature tx. In another variant, a sensor array may comprise multiple sensor cells wherein at least one of the multiple sensor cells - and in another variant preferably all of the multiple sensor cells - is/are designed such that such cell/s may mainly be sensitive to different analytes under different operating conditions such as under different temperatures. In such specific embodiment, each of such cell/s may be provided with an individual heater. In other embodiments, all cells may be heated by the same heater.

However, the chemical sensor may be based on one of the following measurement principles without limitation: A chemomechanical principle, in which a mass change upon absorption is transformed into a surface acoustic wave, or into a cantilever resonance, for example. Alternatively, there may be thermal sensing concepts applied, e.g. by making use of pellistors which may serve as a catalytic thermal sensor in which heat is generated or consumed during combustion. Alternatively, the chemical sensor may rely on optical detection, such as in form of a microspectrometer, or an NDIR, or may make use of electrochemical reactions such as being enabled by solid state electrolytes in combination with voltammetric, potentiometric, or conductometric measurement principles. Chemiresistors may also be used, such as conducting and carbon-loaded polymers, preferably in a low-temperature arena, or, metal-oxide sensors such as tin oxide, tungsten oxide, gallium oxide, indium oxide, zinc oxide, titanium oxide, which preferably may be applied in a high-temperature arena. ISFET (ion-selective FET) may also be used, as well as chemocapacitors wherein it is preferred to use a polymer as active material.

In case the chemical sensor is embodied as a sensor array, the individual sensor cells may preferably be embodied as discrete sensor cells arranged on a common conductor board. In a different embodiment, the sensor cells may be represented by multiple chips the sensing structures are integrated in. Here, each individual chip may be packaged, i.e. encapsulated, and arranged on a conductor board. In an alternative arrangement, such multiple sensor chips may comprise a common package, such that these chips are encapsulated by a common encapsulation, which package finally is arranged on the conductor board. In a further embodiment, the sensor cells are monolithically integrated into a common sensor chip with a common substrate for all sensor cells. Such monolithic sensor chip may still be encapsulated and be arranged on and electrically connected to a conductor board of the portable electronic device.

Calibration data may be stored in a nonvolatile memory of the chemical sensor. Such calibration data may be applied to the sensor signal for compensating for drifts in the sensor signal, for example.

The chemical sensor may be adapted to supply a measurement tuple comprising a set of tuple elements with each tuple element of the set of tuple elements being assigned to a dedicated cell of the chemical sensor and/or to a dedicated operating condition of the chemical sensor or of a cell of the chemical sensor. In the measurement tuple, after a measurement, each tuple element may hold a value measured with respect to the assigned sensor cell and/or the assigned operating condition during an odour and/or gas measurement. In case, each sensor cell and/or each operating condition is embodied for being mainly sensitive to an analyte assigned, each measurement tuple may also be interpreted as an indicator of the presence and possibly a concentration of such analyte. This means that the chemical sensor provides a measurement result in form of a tuple with each tuple element representing the result of an individual sensor cell in case of a sensor array which sensor cell is mainly sensitive to one of the analytes. Hence, the measurement result of the chemical sensor with respect to a measurement - i.e. a measurement taken simultaneously by all sensor cells or taken within a reasonable time frame for belonging to the same measurement scenario - is assembled in a data structure referred to as measurement tuple and is supplied to an evaluation unit as will be explained later on.

Preferably, each measurement tuple element provides a normalized value. This means, that values measured by the cells, for example, are normalized with respect to a norm. Such norm may be, for example, the value measured by one of the cells. Accordingly, each value measured is divided by the value measured by one of the sensor cells. Given that the value of each measurement is dependent on a magnitude/strength of the gas or odour, such magnitude dependence may not be preferred in comparing the measurement tuple to the one or more reference tuples as will be explained later on. In an application for identifying an odour, for example, comparing a strong dose of the subject odour as measured to a smaller dose of the subject odour as a reference would not lead to a match in the comparison and as such not lead to the desired result. Hence, it is preferred that all tuple elements, be it the ones of the measurement tuple and be it the ones of the one or more reference tuples contain normalized values and all make use of a common norm. Then, a tuple may also be considered as a normalized vector.

It is preferred that the chemical sensor may be assigned a processing unit for supporting the assembly of the measurement tuple. Such processing unit may be a processing unit dedicated to the chemical sensor unit, or may be a processing unit of the portable electronic device that is made available for the subject task. Preferably, the measurement tuple is supplied in combination with one or more of the following data: a chemical sensor unit identifier, a time stamp, global positioning information referring to the location of the portable electronic device at the time of measurement, etc. With such additional information, the odour and/or gas identification request may be better tracked. A tuple in general may also be referred to as vector.

The database is a database containing a set of reference tuples with each reference tuple representing an odour and/or gas and comprising a set of tuple elements and an identifier for the odour and/or gas represented by the concerned reference tuple. For a given reference tuple its elements are assigned to dedicated cells of the chemical sensor and/or dedicated operating conditions of the chemical sensor or of the cell of the chemical sensor, and as a result possibly to the various analytes the chemical sensor mainly is sensitive to. And each tuple element holds a value characteristic for being sensed by the dedicated cell of the chemical sensor and/or under the dedicated operating condition of the chemical sensor or of the cell of the chemical sensor in the presence of the odour and/or gas represented by the concerned reference tuple. Such value may preferably be a normalized value. Hence, a reference tuple represents an odour and/or gas and specifically defines an odour and/or gas with respect to the set of analytes the chemical sensor is sensitive to. For example, if the chemical sensor is mainly sensitive to nine different analytes, a reference tuple representing the odour of a "tulip" may contain nine tuple elements with each tuple element providing a value reflecting the presence, absence, or partial presence of the assigned analyte out of the nine analytes the chemical sensor mainly is sensitive to. Each reference tuple is tagged by an identifier identifying the odour and/or gas which the subject reference tuple represents. The identifyer may be embodied as a numeral identifier which, for example, may generally be used in a classification system for odours and/or gases. Or it may be a verbal descriptor of the odour, such as "rose", for example. Or it may be any other kind of identifier for identifying odour and/or gases.

The database containing reference tuples for odours and/or gases may be built by a database provider who may have measured odours and/or gases by means of a portable electronic device of the same kind, i.e. preferably containing an identical chemical sensor as is used for measuring odours and/or gas and assembling the associate measurement tuple later on by a user of the portable electronic device, and as such being sensitive to the same analytes, and preferably showing the same sensitivity for each analyte. Each odour and/or gas measured may be flagged by the provider with an identifier. For example, the provider may hold the portable electronic device close to a rose, trigger a measurement, have the measurement values stored in form of a tuple and may have assigned, for example, via an input on a keyboard or a touchscreen of the portable electronic device a written description of the odour and/or gas as identifier. The measurement tuple and the assigned identifier may be transmitted to the database and be stored there, e.g. via a wireless link in case the portable electronic device has a wireless communication capability. In a different scenario, users, and in another embodiment users registered with the database provider, may add reference tuples to the database by this enhancing the database to a powerful source of odour and/or gas reference tuples.

The underlying idea is to support a user of a portable electronic device in identifying an odour and/or gas that the user possibly may smell and/or a chemical composition that the user may not smell and which is measured by the chemical sensor of the users portable electronic device. For this purpose, the measurement tuple may be compared to one or more reference tuples from the database for identifying the odour and/or gas measured. In case, for example, the odour measured is the odour of a rose, a comparison of the measurement tuple with reference tuples from the data base may more or less match with one of the reference tuples which reference tuple represents the odour of a rose and which reference tuple accordingly is tagged as "rose". This tag/identifier then is returned as odour identified by the evaluation unit based on a comparison of the measurement tuple with one or more reference tuples of the database.

The comparison in the evaluation unit may include a determination of a deviation between each measurement tuple element and its counterpart reference tuple element. In this context, a measurement tuple element and its counterpart reference tuple element are linked in that they are assigned to the same cell of the chemical sensor and/or to the same operating condition of the chemical sensor or of the cell of the chemical sensor. Hence, both tuple elements indicate a value, an in particular a normalized value, of e.g. the absence, presence or partial presence of the assigned analyte which is measured on the one hand, and which is provided within the reference tuple for characterizing the presence of the assigned analyte in the subject odour on the other hand. Since a tuple generally contains at least two tuple elements, there may be at least two deviations determined in the evaluation process. In case normalization is applied to the values of the measurement and reference tuple elements, at least three tuple elements are required for allowing comparison of at least two of the tuple elements of the measurement and the reference tuple since one of the tuple elements is used for normalization. Hence, it may depend on a result of the at least two deviations if or if not an odour and/or gas identifier is returned. For example, each deviation may be compared to an assigned threshold, and the identifier of the reference tuple under investigation is returned in case a number n of deviations is below the assigned thresholds. This means that at least n analytes need to closely match a typical concentration of the subject analytes as is manifested in the subject reference tuple for the subject odour/gas.

In a variant, the identifier of two or more measurement tuples may be returned. This may be the case, if no single reference tuple unambiguously matches the measurement tuple. In such case, the two or more reference tuples coming closest to the measurement tuple are selected for an odour and/or gas suggestion. For example, the identifiers of n reference tuples with a lowest accumulation value of all tuple element deviations may be returned. In another embodiment, a statement as to a likelihood of the different odours and/or gases suggested may be returned in combination with the identifiers. In another scenario, a void identifier may be returned in case no single reference tuple has sufficiently matched the measurement tuple.

Preferably, the evaluation unit may apply some sort of pattern recognition for comparing the measurement tuple to one or more reference tuples. In this context, but also in a general context, a comparison between tuples and/or graphical representations of the tuples may include a comparison of a deviation between the measurement tuple and a reference tuple to a variable threshold, or, in another embodiment, to a threshold corridor/range within which the measurement tuple is expected to fall for compliance. Preferably all the reference tuples are compared to the measurement tuple in order to increase chances of a match. Smart algorithms may be applied for performing the measurement step.

For example, only one measurement tuple element may be compared to all corresponding reference tuple elements. In a next step, only the x reference tuples containing the x reference tuple elements that came closest to the first measurement tuple element are selected for a comparison with the second measurement tuple element.

With respect to the allocation of components of the odour and/or gas identification system, the following embodiments are preferred:

In a first aspect, the database and the evaluation unit are arranged remote from the portable electronic device. In view of the database claiming considerable storage space a server / a server system / a cloud system may preferably be used for providing the database. The evaluation unit may be represented by processing power at the same system remote from the portable electronic device or on a different system remote from the portable electronic device. A service provider may offer services to users or subscribers of identifying odours and/or gases received in form of measurement tuples. A user may register with the provider and receive the odour and/or gas identifiers in return for a measurement tuple sent. Any transmission of a measurement tuple and/or of an identifier return is performed via a wireless link, e.g. based on a UMTS or a GPRS standard. In another embodiment, the provider may return the identifier via SMS.

In a different aspect, the database is remote from the portable electronic device, however, the evaluation unit is arranged in the portable electronic device. The evaluation unit may be embodied as hardware or firmware or as software on a general purpose processor unit of the portable electronic device. In this scenario, the electronic portable device requests one or more reference tuples from the database for comparing the measurement tuple to locally, i.e. on the portable electronic device. Again, a wireless link is used for requesting/fetching and receiving reference tuples from the database.

Hence, any portable electronic device such as a mobile phone, and in particular a smart phone, a handheld computer, an electronic reader, a tablet computer, a game controller, a pointing device, a photo or a video camera, or a computer peripheral - which listing is not limited - may in addition to its original function support the chemical and/or odour and/or gas identification as to its environment. Such portable electronic device as a result may primarily be designed for computing and/or telecommunication and/or other tasks in the IT arena, and now may be enhanced by the function of providing chemical information as to its environment. The user may learn about chemical substances, compositions and/or odours present in the devices surroundings, and may use, transmit or else further analyse such information. For the reason that such portable electronic device typically includes interfaces to a remote infrastructure, such information may also be transmitted elsewhere and used elsewhere. In an alternative, the user himself/herself may benefit from the information provided by the chemical sensor in that actions may be taken in response to detected gases and/or odours, including but not limited to toxic substances and compounds such as CO. An alarm may be triggered once such compound is identified in form of a return of the "CO" identifier, for example. Other measurements may be taken out of pure interest, such as, for example, the measurement of the odour of a flower or the odour of a drink. In response to such measurement, the identifier/name of an odour unknown to the user, such as "sunflower", for example, or "bourbon 10 yrs old", may be displayed to the user.

In another embodiment, there may be at least one additional sensor provided out of a humidity sensor and a temperature sensor. These sensors may help in compensating temperature induced and/or humidity induced signal variations in a signal of the chemical sensor. Preferably the temperature sensor and/or the humidity sensor may be arranged in proximity to the chemical sensor, for example in the same opening of a housing of the portable electronic device.

In a preferred embodiment, the portable electronic device comprises an input unit for triggering an odour and/or gas measurement. Such input may be a key or a touchscreen element, for example. It may enable the user to start a measurement. The user may first arrange the portable electronic device close to a source of odour and/or gas and then trigger the measurement. In a different embodiment the chemical sensor unit is adapted to periodically supply measurement tuples over time, for example at regular intervals. This operational mode may be advantageous when the user may stay in a polluted environment, for example.

In general, any measurement finalized by the chemical sensor unit may initiate a transfer of the measurement tuple to the evaluation unit and may initiate an evaluation there.

In a preferred embodiment, there may be multiple databases provided by different providers or users, and the portable electronic device may comprise a selector unit for offering a selection of one or more of a multitude of databases to the user for applying reference tuples from. The selector unit may be built in form of a key or a touchscreen, for example. There may be scenarios in which official or commercial provider databases may be trusted more than private user databases such a selection is desirable. This may be true, for example, for databases containing reference vectors representing vapours of organic solvents. An associate service provider may have collected and tagged the signal vectors under controlled conditions.

According to a further aspect of the present invention, methods are provided for identifying an odour and/or gas. An odour and/or gas is measured with a portable electronic device comprising a chemical sensor unit, the chemical sensor unit being sensitive to different analytes. A measurement tuple is supplied comprising a set of tuple elements with each tuple element of the set of tuple elements providing a value measured by a dedicated cell of the chemical sensor and/or under a dedicated operating condition of the chemical sensor or of a cell of the chemical sensor during an odour and/or gas measurement. The measurement tuple is compared to one or more reference tuples with each reference tuple representing an odour and/or gas and comprising a set of tuple elements and an identifier for the odour and/or gas represented by the reference tuple. Each tuple element of the set of tuple elements provides a value characteristic for being sensed by the dedicated cell of the chemical sensor and/or under the dedicated operating condition of the chemical sensor or of the cell of the chemical sensor in the presence of the odour and/or gas represented by the concerned reference tuple. Subject to a result of the comparison of one or more odour and/or gas identifiers are returned to the portable electronic device. According to another aspect of the present invention, a computer program medium, comprising computer program code means is provided for implementing the following steps when executed on a processing unit: A measurement tuple is received from a chemical sensor unit and comprises a set of tuple elements with each tuple element of the set of tuple elements provides a value measured by a dedicated cell of the chemical sensor and/or under a dedicated operating condition of the chemical sensor or of a cell of the chemical sensor during an odour and/or gas measurement. The measurement tuple is compared to one or more reference tuples with each reference tuple representing an odour and/or gas and comprising a set of tuple elements and an identifier for the odour and/or gas represented by the reference tuple. Each tuple element of the set of tuple elements provides a value characteristic for being sensed by the dedicated cell of the chemical sensor and/or under the dedicated operating condition of the chemical sensor or of the cell of the chemical sensor in the presence of the odour and/or gas represented by the concerned reference tuple. Subject to a result of the comparison one or more odour and/or gas identifiers are returned.

Other advantageous embodiments are listed in the dependent claims as well as in the description below.

All described embodiments similarly pertain to the devices, the methods, and the computer program product. Synergetic effects may arise from different combinations of the embodiments although they might not be described in detail.

### Brief Description of the Drawings

The embodiments defined above and further aspects, features and advantages of the present invention can also be derived from the examples of embodiments to be described hereinafter and are explained with reference

The embodiments defined above and further aspects, features and advantages of the present invention can also be derived from the examples of embodiments to be described hereinafter and are explained with reference to the drawings. In the drawings the figures illustrate in
FIG. 1 a usage scenario with a mobile phone according to an embodiment of the present invention,
FIG. 2 a block diagram of a portable electronic device according to an embodiment of the present invention,
FIG. 3 a functional diagram of an odour and/or gas identification system according to an embodiment of the present invention,
FIG. 4 a flow diagram representing a method according to an embodiment of the present invention,
FIG. 5 a top view on a chemical sensor unit chip according to an embodiment of the present invention,
FIG. 6 an illustration of a comparison step in a method according to an embodiment of the present invention, and
FIG. 7 an illustration of two different measurement tuples as used in an embodiment of the present invention.

### Modes for Carrying Out the Invention

Same or similar elements are referred to by the same reference numerals across all Figures.

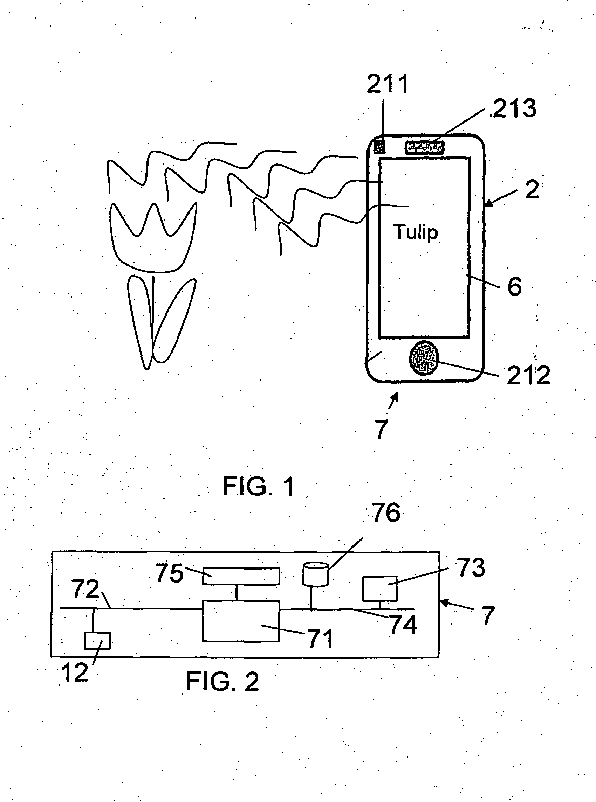

Figure 1 illustrates a usage scenario with a mobile phone 7 according to an embodiment of the present invention. Apart from a standard microphone as an input device which microphone is arranged in an opening 212 of a front wall 21 of a housing 2, a chemical sensor is arranged in another opening 211 of the front wall 21, which substances respectively, is sensed by the chemical sensor. By means of a database and an evaluation unit -the sensed odour is compared to reference odours and is finally identified as an odour from tulips. This result is displayed on a display 6 of the mobile phone 7.

Figure 2 shows a schematic hardware oriented block diagram of a portable electronic device in form of a mobile phone 7. A microprocessor 71 is connected via electrical conductors 72 to a chemical sensor 12, which electrical conductors 72 specifically may be conductors of a flexible printed circuit board. The chemical sensor 12 contains signal processing capability in order to output an odour and/or gas tuple measured. A routine for analysing the odour supplied by the chemical sensor 12 in form of a measurement tuple may be executed by an evaluation unit. A hardware of the evaluation unit may be represented by the microprocessor 71, and a software of the evaluation unit may be represented by a program element stored in a memory 73 connected to the microprocessor 71 via a bus system 74. In an arrangement, which is not claimed, a database 76 containing reference tuple data may be connected to the microprocessor 71 via the bus system 74, too. A wireless interface 75 may be connected to the microprocessor 71.

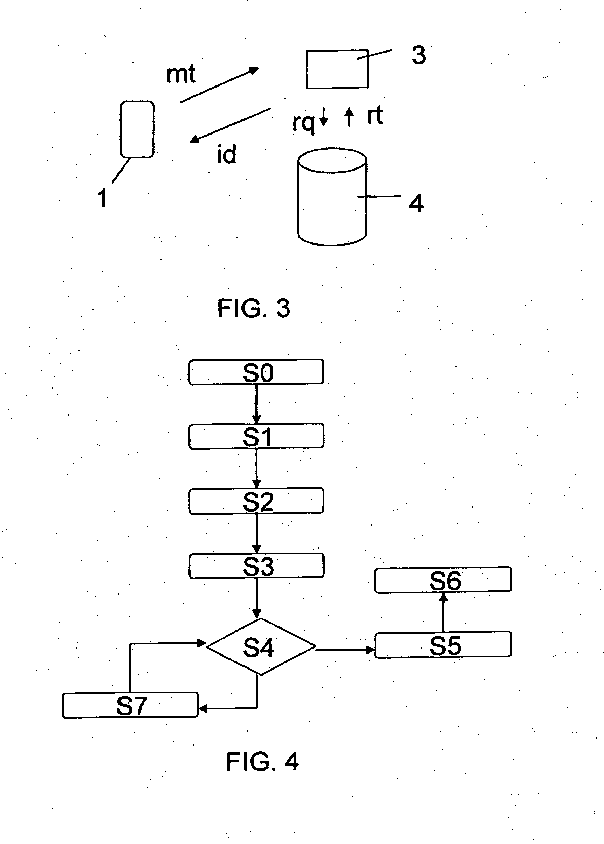

Figure 3 illustrates a functional diagram of an odour and/or gas identification system according to an embodiment of the present invention. In this specific case, the portable electronic device may be embodied as a tablet computer 1 comprising a chemical sensor not further shown in Figure 3. The chemical sensor provides a measurement tuple mt to an evaluation unit 3 remote from the tablet computer 1. The evaluation unit 3 may be embodied in a server hosting a service for odour and/or gas identification. Once the evaluation unit 3 has received a measurement tuple mt, this is taken as a request to start an identification process which is initiated by submit-bodied in a server hosting a service for odour and/or gas identification. Once the evaluation unit 3 has received a measurement tuple mt, this is taken as a request to start an identification process which is initiated by submitting a request rq to a database 4 for reference tuple data. The database 4 is remote from the tablet computer 1 and the evaluation unit 3 and contains storage space for storing reference tuples rt representing odours and/or gases, for example. The reference tuples rt are transmitted to the evaluation unit 3 as requested and are compared to the measurement tuple mt there. Once a match is identified between the measurement tuple mt and one of the reference tuples rt, an identifier id for the odour and/or gas represented by this specific reference tuple rt is returned to the tablet computer 1.

Figure 4 illustrates a flow diagram representing a method according to an embodiment of the present invention. In a first step S0, a measurement of an odour and/or gas is initiated by a user of a portable electronic device containing a chemical sensor. In step S1, the measurement is performed, and the measurement results are assembled in a data structure denoted as measurement tuple and having a digital format. In step S2, the measurement tuple is sent to an evaluation unit located remote from the portable electronic device. In step S3, the evaluation unit sets a counter i to zero. In step S4, the evaluation unit which may together with a data-base reside in a server, retrieves the i^{th} reference tuple from the database and compares the measurement tuple to the i^{th} reference tuple. In case the measurement tuple matches the i^{th} reference tuple (Y), an identifier of the subject reference tuple is returned by the evaluation unit to the portable electronic device in step S5. In step S6, the identifier may be displayed on a display of the portable electronic device. In case the measurement tuple does not match the i^{th} reference tuple (N), the reference tuple counter i is increased in step S7, and in step S4 the measurement tuple is compared to the next reference tuple from the database.

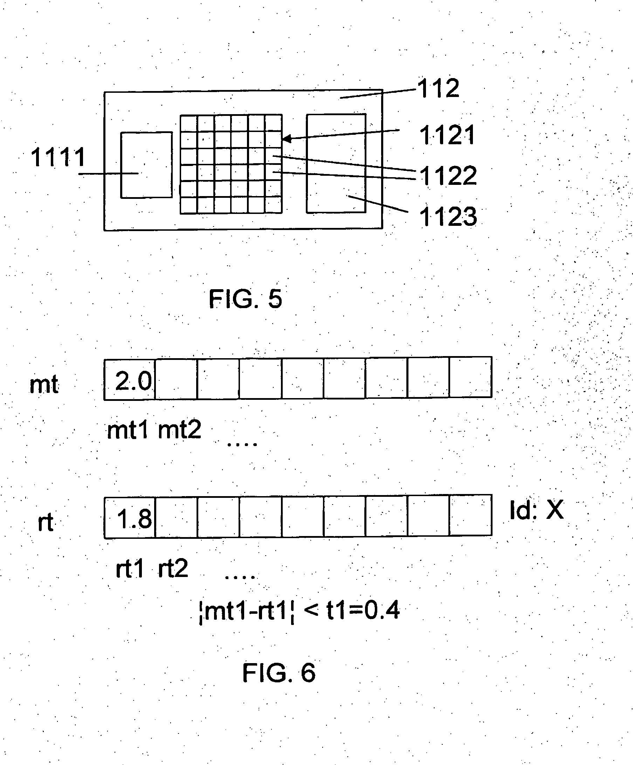

Figure 5 illustrates a top view on a chip 112 representing a chemical sensor as is used in an embodiment of the present invention. The chip 112 comprises a chemical sensor structure 1121 which takes the shape of a sensor array comprising multiple symbolic sensor cells 1122, in the present example, thirty six sensor cells 1122. In addition a humidity sensitive structure 1111 is arranged next to the chemical sensor structure 1121, and electronic circuitry 1123 is integrated into the chemical sensor chip 112 which electronic circuitry 112 is responsible for applying a humidity compensation of the signal supplied by the chemical sensor structure 1121, for linearizing and A/D converting the sensor signal, and for building the measurement tuple, for example.

FIG. 6 illustrates an illustration of a comparison step in a method according to an embodiment of the present invention. A sample measurement tuple mt is depicted containing nine measurement tuple elements mt1 ...mx ... mt9, each tuple element holding a value wherein only the value 2.0 of the first measurement tuple element mt1 is shown for illustration purposes. A sample reference tuple rt is depicted containing nine reference tuple elements rt1 ....rtx ... rt9, each tuple element holding a value wherein only the value 1.8 of the first reference tuple element rt1 is shown for illustration purposes. The reference tuple rt represents odour id=X. The first measurement tuple element mt1 is assigned to a first sensor cell of the chemical sensor which first sensor cell is mainly sensitive to a first analyte. The corresponding first reference tuple element rt1 is assigned to the same sensor cell and hence, provides a value as to the same first analyte. The value of the first measured tuple element mt1 is compared to the first reference tuple element rt1 by determining a deviation mt1-rt1 which deviation is compared to a first threshold t1, which threshold is the present example is set to 0.4. The interpretation of the foregoing may be as follows: A concentration of 2.0 of the first analyte was measured. The reference odour named "X" typically shows a concentration of this analyte of 1.8. As long as the deviation of the measured concentration from the reference concentration is less than 0.4, the measured odour and/or gas may be "X" subject to a concentration of the other analytes measured which concentrations may be compared to the reference concentrations in the same way.

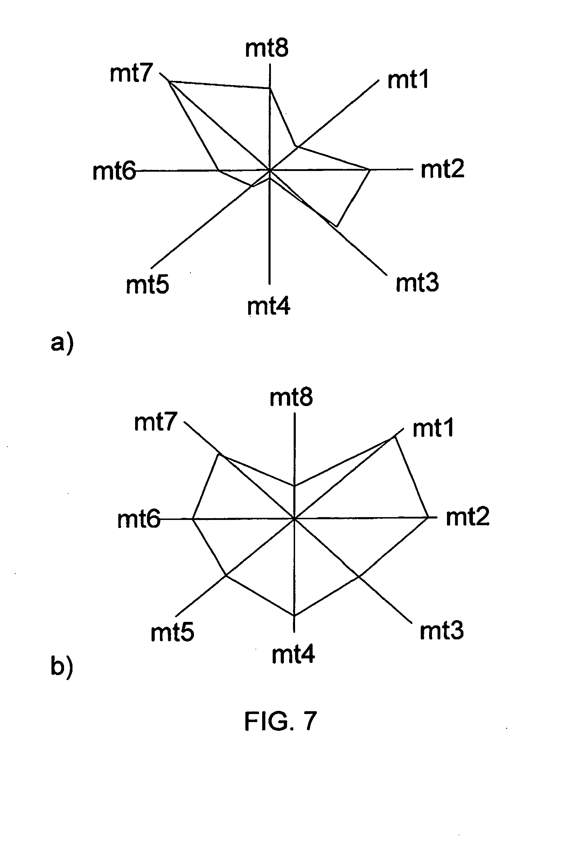

FIG. 7 illustrates in diagrams a) and b) two different measurement tuples mt as used in an embodiment of the present invention. Each measurement tuple mt is sensed by a chemical sensor being sensitive to eight analytes, such that a measurement tuple mt contains eight tuple elements mt1 ... mt8. The measurement tuple mt according to diagram 7a) represents a measurement of a first gas/odour, and the measurement tuple mt according to diagram 7b) represents a measurement of a second gas/odour. Each measurement tuple mt is represented by a spider diagram with eight axes, and with the value of each tuple element mt1 ... mt8 being reflected on the respective axis crossing. The values as shown may be normalized values in order for a specific odour to result in the same spider diagram every time independent of a strength of the odour. For example, each measured value may be converted into a normalized value by dividing the value by the value measured for the first sensor cell, i.e. mt1. Preferably, normalized patterns are compared to each other. Other approaches of normalizations ma be applied, for example, a normalization with respect to an area of the spider diagram, etc. Such spider diagram measurement tuple representation may support graphical pattern recognition. The reference tuples may also be stored as graphical representations in form of spider diagrams. In a best match algorithm, the reference pattern that matches the measurement pattern best is identified and its tag is returned.

While there are shown and described presently preferred embodiments of the invention, it is to be distinctly understood that the invention is not limited thereto but may be otherwise variously embodied and practised within the scope of the following claims.

## Claims

1. Odour and/or gas identification system, comprising
a portable electronic device comprising a chemical sensor (12) being sensitive to different analytes and being adapted for supplying a measurement tuple (mt) comprising a set of tuple elements with each tuple element (mtx) of the set of tuple elements (mtx) providing a value measured during an odour and/or gas measurement by a dedicated cell of the chemical sensor and/or under a dedicated operating condition of the chemical sensor or of a cell of the chemical sensor;
a database (76,4), wherein the database is arranged remote from the portable electronic device, containing a set of reference tuples (rt) with each reference tuple (rt) representing an odour and/or gas and comprising
- a set of tuple elements with each tuple element (rtx) of the set of tuple elements (rtx) providing a value characteristic for being sensed by the dedicated cell of the chemical sensor and/or under the dedicated operating condition of the chemical sensor or of the cell of the chemical sensor in the presence of the odour and/or gas represented by the concerned reference tuple (rt), and
- an identifier (id) for the odour and/or gas represented by the concerned reference tuple (rt);
an evaluation unit (71,3) for comparing the measurement tuple (mt) to one or more reference tuples (rt) out of the set of reference tuples (rt) and subject to a result of the comparison returning one or more odour and/or gas identifiers (id) to the portable electronic device,
wherein the portable electronic device is one of:
a mobile phone (7),
a handheld computer,
an electronic reader,
a tablet computer (1),
a game controller,
a pointing device,
a photo or a video camera,
a computer peripheral,
wherein the evaluation unit (3) is arranged remote from the portable electronic device,
wherein the electronic portable device is adapted to send the measurement tuple (mt) to the evaluation unit (3),
wherein the evaluation unit (3) is adapted to receive the measurement tuple (mt) from the electronic device and is adapted to send the one or more odour and/or gas identifiers (id) to the portable electronic device, and
wherein a wireless link is used for sending the measurement tuple (mt) and/or the one or more odour and/or gas identifiers (id).

2. Odour and/or gas identification system, comprising
a portable electronic device comprising a chemical sensor (12) being sensitive to different analytes and being adapted for supplying a measurement tuple (mt) comprising a set of tuple elements with each tuple element (mtx) of the set of tuple elements (mtx) providing a value measured during an odour and/or gas measurement by a dedicated cell of the chemical sensor and/or under a dedicated operating condition of the chemical sensor or of a cell of the chemical sensor;
a database (76,4), wherein the database is arranged remote from the portable electronic device, containing a set of reference tuples (rt) with each reference tuple (rt) representing an odour and/or gas and comprising
- a set of tuple elements with each tuple element (rtx) of the set of tuple elements (rtx) providing a value characteristic for being sensed by the dedicated cell of the chemical sensor and/or under the dedicated operating condition of the chemical sensor or of the cell of the chemical sensor in the presence of the odour and/or gas represented by the concerned reference tuple (rt), and
- an identifier (id) for the odour and/or gas represented by the concerned reference tuple (rt);
an evaluation unit (71,3) for comparing the measurement tuple (mt) to one or more reference tuples (rt) out of the set of reference tuples (rt) and subject to a result of the comparison returning one or more odour and/or gas identifiers (id) to the portable electronic device,
wherein the portable electronic device is one of:
a mobile phone (7),
a handheld computer,
an electronic reader,
a tablet computer (1),
a game controller,
a pointing device,
a photo or a video camera,
a computer peripheral,
wherein the portable electronic device contains the evaluation unit (71),
wherein the electronic portable device is adapted to fetch one or more reference tuples (rt) from the database (4) for comparing the measurement tuple (mt) to, and
wherein a wireless link is used for fetching the one or more reference tuples (rt) from the database (4).

3. System according to any one of the preceding claims,
comprising a display (6) for displaying the one or more odour and/or gas identifiers (id), and
in particular wherein the portable electronic device comprises the display (6).

4. System according to any one of the preceding claims,
wherein the chemical sensor (12) comprises an array of sensor cells (1122), and
in particular wherein each sensor cell (1122) is mainly sensitive to a different one of the analytes the chemical sensor (12) is sensitive to.

5. System according to any one of the preceding claims,
wherein the portable electronic device comprises at least one of
- a temperature sensor for compensating temperature dependent signal variations in a signal of the chemical sensor (12), and
- a humidity sensor (1111) for compensating humidity dependent signal variations in a signal of the chemical sensor (12).

6. System according to any one of the preceding claims,
wherein the portable electronic device comprises an input unit for triggering an odour and/or gas measurement.

7. System according to any one of the preceding claims,
wherein the chemical sensor (12) is adapted to continuously supply measurement tuples (mt) over time.

8. System according to anyone of the preceding claims,
wherein the portable electronic device comprises a selector unit for selecting one or more of a multitude of databases (76,4) for applying reference tuples (rt) from.

9. System according to anyone of the preceding claims,
wherein the chemical sensor (12) is adapted to supply the measurement tuple (mt) in combination with a chemical sensor identifier, and in particular in combination with a time stamp.

10. Portable device for use in an odour and/or gas identification system according to any one of the preceding claims.

11. Method for identifying an odour and/or gas, comprising the steps of
measuring an odour and/or gas with a portable electronic device comprising a chemical sensor (12), the chemical sensor (12) being sensitive to different analytes, which portable electronic device is one of a mobile phone (7), a handheld computer, an electronic reader, a tablet computer (1), a game controller, a pointing device, a photo or a video camera, a computer peripheral,
supplying a measurement tuple (mt) comprising a set of tuple elements with each tuple element (mtx) of the set of tuple elements (mtx) providing a value measured during an odour and/or gas measurement by a dedicated cell of the chemical sensor and/or under a dedicated operating condition of the chemical sensor or of a cell of the chemical sensor;
the electronic portable device sending the measurement tuple (mt) via a wireless link to an evaluation unit (3) arranged remote from the portable electronic device,
- the evaluation unit (3) receiving the measurement tuple (mt) from the electronic device and comparing the measurement tuple (mt) to one or more reference tuples (rt) of a database, wherein the database is arranged remote from the portable electronic device, with each reference tuple (rt) representing an odour and/or gas and comprising
- a set of tuple elements with each tuple element (rtx) of the set of tuple elements (rtx) providing a value characteristic for being sensed by the dedicated cell of the chemical sensor and/or under the dedicated operating condition of the chemical sensor or of the cell of the chemical sensor in the presence of the odour and/or gas represented by the concerned reference tuple (rt), and
- an identifier (id) for the odour and/or gas represented by the reference tuple (rt); and
the evaluation unit (3) returning one or more odour and/or gas identifiers (id) via the wireless link to the portable electronic device subject to a result of the comparison.

12. Method for identifying an odour and/or gas, comprising the steps of
measuring an odour and/or gas with a portable electronic device comprising a chemical sensor (12), the chemical sensor (12) being sensitive to different analytes, which portable electronic device is one of a mobile phone (7), a handheld computer, an electronic reader, a tablet computer (1), a game controller, a pointing device, a photo or a video camera, a computer peripheral,
supplying a measurement tuple (mt) comprising a set of tuple elements with each tuple element (mtx) of the set of tuple elements (mtx) providing a value measured during an odour and/or gas measurement by a dedicated cell of the chemical sensor and/or under a dedicated operating condition of the chemical sensor or of a cell of the chemical sensor;
an evaluation unit (71) contained in the portable electronic device fetching one or more reference tuples (rt) from a database (4) via a wireless link for comparing the measurement tuple (mt) to,
comparing the measurement tuple (mt) to the one or more reference tuples (rt) fetched from the database, wherein the database is arranged remote from the portable electronic device, with each reference tuple (rt) representing an odour and/or gas and comprising
- a set of tuple elements with each tuple element (rtx) of the set of tuple elements (rtx) providing a value characteristic for being sensed by the dedicated cell of the chemical sensor and/or under the dedicated operating condition of the chemical sensor or of the cell of the chemical sensor in the presence of the odour and/or gas represented by the concerned reference tuple (rt), and
- an identifier (id) for the odour and/or gas represented by the reference tuple (rt); and
returning one or more odour and/or gas identifiers (id) to the portable electronic device subject to a result of the comparison.

13. Method for identifying an odour and/or gas according to claim 11 or claim 12,
wherein the measurement tuple (mt) is compared to the one or more reference tuples (rt) by means of determining a deviation between measurement tuple elements (mtx) and counterpart reference tuple elements (rtx) with a measurement tuple element (mtx) and its counterpart reference tuple element (rtx) being assigned to the same cell of the chemical sensor and/or to the same operating condition of the chemical sensor or of the cell of the chemical sensor, and
wherein the identifier (id) of a reference tuple (rt) compared to the measurement tuple (mt) is returned subject to the deviations determined, and
in particular wherein the tuple elements (mtx, rtx) to be compared comprise normalized values.

14. Method according to claim 13, wherein each deviation is compared to a threshold (tx) assigned, and
wherein the identifier (id) of a reference tuple (rt) compared to the measurement tuple (mt) is returned in case a number n of deviations is below the assigned thresholds (tx).

15. Computer program element comprising computer program code means for implementing the hollowing steps when executed on a processing unit of one of a mobile phone (7), a handheld computer, an electronic reader, a tablet computer (1), a game controller, a pointing device, a photo or a video camera, a computer peripheral: receiving a measurement tuple (mt) from a chemical sensor unit (12) and comprising a set of tuple elements with each tuple element of the set of tuple elements providing a value measured by a dedicated cell of the chemical sensor and/or under a dedicated operating condition of the chemical sensor or of a cell of the chemical sensor during an odour and/or gas measurement;
fetching one or more reference tuples (rt) via a wireless link from a database, wherein the database is arranged remote from the portable electronic device, with each reference tuple (rt) representing an odour and/or gas and comprising
- a set of tuple elements with each tuple element (rtx) of the set of tuple elements (rtx) providing a value characteristic for being sensed by the dedicated cell of the chemical sensor and/or under the dedicated operating condition of the chemical sensor or of the cell of the chemical sensor in the presence of the odour and/or gas represented by the concerned reference tuple (rt), and
- an identifier (id) for the odour and/or gas represented by the reference tuple (rt) ; comparing said measurement tuple (mt) to said one or more reference tuples (rt);
and returning one or more odour and/or gas identifiers (id) subject to a result of the comparison.

## Patentansprüche

1. Geruchs- und/oder Gaserkennungssystem, umfassend
ein tragbares elektronisches Gerät, umfassend einen chemischen Sensor (12) empfindlich auf verschiedene Analyten und geeignet zum Bereitstellen eines Messtupels (MT) umfassend einen Satz von Tupelelementen, wobei jedes Tupelelement (MTX) aus dem Satz von Tupelelemente (MTX) einen Wert bereitstellt, der während einer Geruchs- und/oder Gasmessung mit einer dedizierte Zelle des chemischen Sensors und/oder unter einem dedizierten Betriebszustand des chemischen Sensors oder einer Zelle des chemischen Sensors, gemessen wurde;
eine Datenbank (76,4), die entfernt vom tragbaren elektronischen Gerät angeordnet ist, enthaltend einen Satz von Referenztupeln (rt), wobei jedes Referenztupel (rt) einen Geruch und/oder ein Gas darstellt und enthält
- einen Satz von Tupelelementen, wobei jedes Tupelelement (RTX) des Satzes von Tupelelementen (RTX) einen Wert bereitstellt, der charakteristisch ist für die Messung mit der dedizierten Zelle des chemischen Sensors und/oder unter dem dedizierten Betriebszustand des chemischen Sensors oder einer Zelle des chemischen Sensor in der Gegenwart des Geruchs und/oder Gases dargestellt durch das betreffende Referenztupel (rt), und
- eine Kennung (id) für den Geruch und/oder das Gas welches durch das betreffendene Referenztupel (rt) dargestellt wird;
eine Auswerteeinheit (71,3) zum Vergleichen des Messtupels (MT) mit einem oder mehreren der Referentupel (rt) aus dem Satz der Referenztupel (RT) und abhängig von einem Ergebnis des Vergleichs zum Zurücksenden eines oder mehrerer Geruchs- und/oder Gaskennungen (id) an das tragbare elektronische Gerät,
wobei das tragbare elektronische Gerät eines ist aus:
Mobiltelefon (7),
Handheld-Computer,
elektronisches Lesegerät,
Tablet-Computer (1),
Game-Controller,
Zeigegerät,
Foto- oder Videokamera,
Computer-Peripherie,
wobei die Auswerteeinheit (3) entfernt vom tragbaren elektronischen Gerät angeordnet ist,
wobei das tragbare elektronische Gerät geeignet ist, das Messtupel (mt) an die Auswerteeinheit (3) zu senden,
wobei die Auswerteeinheit (3) geeignet ist, um das Messtupel (MT) vom elektronischen Gerät zu empfangen und geeignet ist, die einen oder mehrere Geruchs- und/oder Gaskennungen (id) an das tragbare elektronische Gerät zu senden, und
wobei eine drahtlose Verbindung zur Übertragung des Messtupels (MT) und/oder der ein oder mehreren Geruchs- und/oder Gaskennungen (ID) verwendet wird.

2. Geruchs- und/oder Gaserkennungssystem, umfassend
ein tragbares elektronisches Gerät, umfassend einen chemischen Sensor (12) empfindlich auf verschiedene Analyten und geeignet zum Bereitstellen eines Messtupels (MT) umfassend einen Satz von Tupelelementen, wobei jedes Tupelelement (MTX) aus dem Satz von Tupelelementen (MTX) einen Wert bereitstellt, der während einer Geruchs- und/oder Gasmessung mit einer dedizierte Zelle des chemischen Sensors und/oder unter einem dedizierten Betriebszustand des chemischen Sensors oder einer Zelle des chemischen Sensors, gemessen wurde;
eine Datenbank (76,4), die entfernt vom tragbaren elektronischen Gerät angeordnet ist, enthaltend einen Satz von Referenztupeln (rt), wobei jedes Referenztupel (rt) einen Geruch und/oder ein Gas darstellt und enthält
- einen Satz von Tupelelementen, wobei jedes Tupelelement (RTX) des Satzes von Tupelelementen (RTX) einen Wert bereitstellt, der charakteristisch ist für die Messung mit der dedizierte Zelle des chemischen Sensors und/oder unter dem dedizierten Betriebszustand des chemischen Sensors oder einer Zelle des chemischen Sensors in der Gegenwart des Geruchs und/oder Gases dargestellt durch das betreffende Referenztupel (rt), und
- eine Kennung (id) für den Geruch und/oder das Gas welches durch das betreffendene Referenztupel (rt) dargestellt wird;
eine Auswerteeinheit (71,3) zum Vergleichen des Messtupels (MT) mit einem oder mehreren der Referentupel (rt) aus dem Satz der Referenztupel (RT) und abhängig von einem Ergebnis des Vergleichs zum Zurücksenden eines oder mehrerer Geruchs- und/oder Gaskennungen (id) an das tragbare elektronische Gerät,
wobei das tragbare elektronische Gerät eines ist aus:
Mobiltelefon (7),
Handheld-Computer,
elektronisches Lesegerät,
Tablet-Computer (1),
Game-Controller,
Zeigegerät,
Foto- oder Videokamera,
Computer-Peripherie,
wobei das tragbare elektronische Gerät die Auswerteeinheit (3) enthält,
wobei das tragbare elektronische Gerät geeignet ist, um ein oder mehrere Referenztupel (RT) von der Datenbank (4) zum Vergleich mit dem Messtupel (mt) abzurufen, und
wobei eine drahtlose Verbindung zum Abrufen der einen oder der mehreren Referenztupel (RT) von der Datenbank (4) verwendet wird.

3. System nach einem der vorhergehenden Ansprüche,
enthaltend eine Anzeige (6) zum Anzeigen der einen oder mehreren Geruchs- und/oder Gaskennungen (id), und
insbesondere wobei das tragbare elektronische Gerät die Anzeige (6) enthält.

4. System nach einem der vorhergehenden Ansprüche,
wobei der chemische Sensor (12) eine Anordnung von Sensorzellen (1122) enthält, und
insbesondere wobei jede Sensorzelle (1122) je hauptsächlich für einen anderen Analyten, aus denen für die der chemischen Sensors (12) empfindlich ist, empfindlich ist.

5. System nach einem der vorhergehenden Ansprüche,
wobei das tragbare elektronische Gerät mindestens eines enthält aus
- einen Temperatursensor zur Kompensation von temperaturabhängigen Signaländerungen in einem Signal des chemischen Sensors (12), und
- einen Feuchtesensor (1111) zur Kompensation von feuchteabhängigen Signaländerungen in einem Signal des chemischen Sensors (12).

6. System nach einem der vorhergehenden Ansprüche,
wobei das tragbare elektronische Gerät eine Eingabeeinheit zum Auslösen einer Geruchs- und/oder Gasmessung enthält.

7. System nach einem der vorhergehenden Ansprüche,
wobei der chemische Sensor (12) geeignet ist, kontinuierlich über die Zeit Messtupel (mt) bereitzustellen.

8. System nach einem der vorhergehenden Ansprüche,
wobei das tragbare elektronische Gerät eine Auswahleinheit zum Auswählen eines oder mehrerer aus einer Vielzahl von Datenbanken (76,4) zum Anwenden von Referenztupeln (RT) daraus enthält.

9. System nach einem der vorhergehenden Ansprüche,
wobei der chemische Sensor (12) geeignet ist, zum Bereitstellen des Messtupels (mt) in Verbindung mit einer chemischen Sensor-Kennung, und insbesondere in Verbindung mit einer Zeitmarke.

10. Tragbares Gerät zur Verwendung in einem Geruchs- und/oder Gaserkennungssystem nach einem der vorhergehenden Ansprüche.

11. Verfahren zur Erkennung eines Geruchs und/oder Gases, umfassend die Schritte:
Messen eines Geruchs und/oder Gases mit einem tragbaren elektronischen Gerät, umfassend einen chemischen Sensor (12) empfindlich auf verschiedene Analyten, wobei das tragbare elektronische Gerät eines ist aus Mobiltelefon (7), Handheld-Computer, elektronisches Lesegerät, Tablet-Computer (1), Game-Controller, Zeigegerät, Foto- oder Videokamera, Computer-Peripherie,
Bereitstellen eines Messtupels (mt) umfassend einen Satz von Tupelelementen, wobei jedes Tupelelement (mtx) aus dem Satz von Tupelelementen (mtx) einen Wert bereitstellt, der während einer Geruchs- und/oder Gasmessung mit einer dedizierte Zelle des chemischen Sensors und/oder unter einem dedizierten Betriebszustand des chemischen Sensors oder einer Zelle des chemischen Sensors, gemessen wurde;
das tragbare elektronische Gerät sendet das Messtupel (mt) über eine drahtlose Verbindung an eine Auswerteeinheit (3), die entfernt vom tragbaren elektronischen Gerät angeordnet ist,
die Auswerteeinheit (3) empfängt das Messtupel (mt) vom tragbaren elektronischen Gerät und vergleicht das Messtupel (mt) mit einem oder mehreren Referenztupeln (rt) einer Datenbank, die entfernt vom tragbaren elektronischen Gerät angeordnet ist, wobei jedes Referenztupel (rt) einen Geruch und/oder ein Gas darstellt und enthält
- einen Satz von Tupelelementen, wobei jedes Tupelelement (RTX) des Satzes von Tupelelementen (RTX) einen Wert bereitstellt, der charakteristisch ist für die Messung mit der dedizierte Zelle des chemischen Sensors und/oder unter dem dedizierten Betriebszustand des chemischen Sensors oder einer Zelle des chemischen Sensors in der Gegenwart des Geruchs und/oder Gases dargestellt durch das betreffende Referenztupel (rt), und
- eine Kennung (id) für den Geruch und/oder das Gas welches durch das betreffendene Referenztupel (rt) dargestellt wird; und
eine Auswerteeinheit (3), die abhängig von einem Ergebnis des Vergleichs, eine oder mehrerer Geruchs- und/oder Gaskennungen (id) über die drahtlose Verbindung an das tragbare elektronische Gerät zurücksendet.

12. Verfahren zur Erkennung eines Geruchs und/oder Gases, umfassend die Schritte:
Messen eines Geruchs und/oder Gases mit einem tragbaren elektronischen Gerät, umfassend einen chemischen Sensor (12) empfindlich auf verschiedene Analyten, wobei das tragbare elektronische Gerät eines ist aus Mobiltelefon (7), Handheld-Computer, elektronisches Lesegerät, Tablet-Computer (1), Game-Controller, Zeigegerät, Foto- oder Videokamera, Computer-Peripherie,
Bereitstellen eines Messtupels (mt) umfassend einen Satz von Tupelelementen, wobei jedes Tupelelement (mtx) aus dem Satz von Tupelelementes (mtx) einen Wert bereitstellt, der während einer Geruchs- und/oder Gasmessung mit einer dedizierte Zelle des chemischen Sensors und/oder unter einem dedizierten Betriebszustand des chemischen Sensors oder einer Zelle des chemischen Sensors, gemessen wurde;
die im tragbaren elektronischen Gerät enthaltene Auswerteeinheit (3) ruft einen oder mehrere Referenztupel (rt) von einer Datenbank (4) über eine drahtlose Verbindung ab um das Messtupel (mt) damit zu vergleichen,
Vergleich des Messtupels (mt) mit dem einen oder mehreren Referenztupel (rt) abgerufen aus der Datenbank, die entfernt vom tragbaren elektronischen Gerät angeordnet ist, wobei jedes Referenztupel (rt) einen Geruch und/oder ein Gas darstellt und enthält
- einen Satz von Tupelelementen, wobei jedes Tupelelement (RTX) des Satzes von Tupelelementen (RTX) einen Wert bereitstellt, der charakteristisch ist für die Messung mit der dedizierte Zelle des chemischen Sensors und/oder unter dem dedizierten Betriebszustand des chemischen Sensors oder einer Zelle des chemischen Sensors in der Gegenwart des Geruchs und/oder Gases dargestellt durch das betreffende Referenztupel (rt), und
- eine Kennung (id) für den Geruch und/oder das Gas welches durch das betreffendene Referenztupel (rt) dargestellt wird; und abhängig von einem Ergebnis des Vergleichs, Zurücksenden einer oder mehrerer Geruchs- und/oder Gaskennungen (id) an das tragbare elektronische Gerät.

13. Verfahren zur Erkennung eines Geruchs und/oder Gases gemäss Anspruch 11 oder 12,
wobei das Messtupel (mt) mit einem oder mehreren Referenztupeln (rt) verglichen wird, indem eine Abweichung zwischen Messtupelelementen (mtx) und ihren entsprechenden Referenztupelelementen (rtx) bestimmt wird, wobei ein Messtupelelement (mtx) und sein entsprechendes Referenztupelelement (rtx) derselben Zelle des chemischen Sensors und/oder dem gleichen Betriebszustand des chemischen Sensors oder der Zelle des chemischen Sensors zugeordnet sind, und
wobei die Kennung (id) eines Referenztupels (rt) verglichen mit dem Messtupel (mt) abhängig von den bestimmten Abweichungen ausgegeben wird, und
insbesondere wobei die zu vergleichenden Tupelelemente (mtx, rtx) normalisierte Werte beinhalten.

14. Verfahren nach Anspruch 13,
wobei jede Abweichung mit einem zugeordneten Schwellenwert (tx) verglichen wird, und
wobei die Kennung (id) eines Referenztupels (rt) verglichen mit dem Messtupel (mt) ausgegeben wird wenn eine Anzahl n von Abweichungen unterhalb der zugeordneten Schwellenwerte (tx) liegt.

15. Computerprogrammelement, enthaltend Computerprogrammcodemittel zur Durchführung der folgenden Schritte, wenn sie auf einer Verarbeitungseinheit eines oder mehrerer Geräte aus Mobiltelefon (7), Handheld-Computer, elektronisches Lesegerät, Tablet-Computer (1), Game-Controller, Zeigegerät, Foto- oder Videokamera, Computer-Peripherie ausgeführt werden:
Empfangen eines Messtupels (mt) von einer chemischen Sensoreinheit (12) und umfassend einen Satz von Tupelelementen, wobei jedes Tupelelement, aus dem Satz von Tupelelementen, einen Wert bereitstellt, der während einer Geruchs- und/oder Gasmessung mit einer dedizierten Zelle des chemischen Sensors und/oder unter einem dedizierten Betriebszustand des chemischen Sensors oder einer Zelle des chemischen Sensors, gemessen wurde;
Abrufen eines oder mehrerer Referenztupel (rt) über eine drahtlose Verbindung von einer Datenbank, die entfernt vom tragbaren elektronischen Gerät angeordnet ist, wobei jedes Referenztupel (rt) einen Geruch und/oder ein Gas darstellt und enthält
- einen Satz von Tupelelementen, wobei jedes Tupelelement (rtx) des Satzes von Tupelelementen (rtx) einen Wert bereitstellt, der charakteristisch ist für die Messung mit der dedizierten Zelle des chemischen Sensors und/oder unter dem dedizierten Betriebszustand des chemischen Sensors oder einer Zelle des chemischen Sensors in der Gegenwart des Geruchs und/oder Gases dargestellt durch das betreffenden Referenztupel (rt), und
- eine Kennung (id) für den Geruch und/oder das Gas welches durch das betreffendene Referenztupel (rt) dargestellt wird;
Vergleichen des Messtupel (MT) mit einem oder mehreren der Referenztupel (rt);
und Rückgabe einer oder mehrerer Geruchs- und/oder Gaskennungen (id) abhängig von einem Ergebnis des Vergleichs.

## Revendications

1. Système d'identification d'odeur et/ou de gaz, comprenant
un dispositif électronique portable comprenant un capteur chimique (12) sensitif à des différents analytes et étant adapté à fournir un tuple de mesures (mt) comprenant un ensemble d'éléments de tuple avec chaque élément de tuple (mtx) de l'ensemble d'éléments de tuple (mtx) donnant une valeur mesurée pendant une mesure d'odeur et/ou de gaz par une cellule dédié du capteur chimique et/ou dans une condition d'opération dédié du capteur chimique ou d'une cellule du capteur chimique;
une base de données (76, 4), la base de données étant arrangée loin du dispositif électronique portable, contenant un ensemble de tuples de référence (rt), chaque tuple de référence (rt) représentant une odeur et/ou un gaz et comprenant
- un ensemble d'éléments de tuple, chaque élément de tuple (rtx) de l'ensemble d'éléments de tuple (rtx) donnant une valeur caractéristique pour être captée par la cellule dédiée du capteur chimique et/ou dans une condition d'opération dédié du capteur chimique ou de la cellule du capteur chimique en présence de l'odeur et/ou du gaz représenté par le tuple de référence concerné (rt), et
- un identifiant (id) pour l'odeur et/ou le gaz représenté par le tuple de référence concerné (rt);
une unité (71, 3) pour comparer le tuple de mesure (mt) avec un ou plusieurs tuples de référence (rt) de l'ensemble de tuples de référence (rt) et faisant l'objet d'un résultat de la comparaison apportant un ou plusieurs identifiants d'odeur et/ou de gaz (id) au dispositif électronique portable,
le dispositif électronique portable étant un de:
un téléphone mobile (7),
un ordinateur portable,
un lecteur électronique,
un ordinateur tablette (1),
un contrôleur de jeu,
un dispositif de pointage,
un appareil photo ou vidéo,
une périphérique d'ordinateur,
l'unité d'évaluation (3) étant arrangée loin du dispositif électronique portable,
le dispositif électronique portable étant adapté à envoyer le tuple de mesures (mt) à l'unité d'évaluation (3),
l'unité d'évaluation (3) étant adaptée à recevoir le tuple de mesures (mt) du dispositif électronique et étant adaptée à envoyer l'un ou les plusieurs identifiants d'odeur et/ou de gaz au dispositif électronique portable, et
une liaison sans fil étant utilisée pour envoyer le tuple de mesures (mt) et/ou l'un ou les plusieurs identifiants d'odeur et/ou de gaz (id).

2. Système d'identification d'odeur et/ou de gaz, comprenant
un dispositif électronique portable comprenant un capteur chimique (12) sensitif à des différents analytes et étant adapté à fournir un tuple de mesures (mt) comprenant un ensemble d'éléments de tuple avec chaque élément de tuple (mtx) de l'ensemble d'éléments de tuple (mtx) donnant une valeur mesurée pendant une mesure d'odeur et/ou de gaz par une cellule dédié du capteur chimique et/ou dans une condition d'opération dédié du capteur chimique ou d'une cellule du capteur chimique;
une base de données (76, 4), la base de données étant arrangée loin du dispositif électronique portable, contenant un ensemble de tuples de référence (rt), chaque tuple de référence (rt) représentant une odeur et/ou un gaz et comprenant
- un ensemble d'éléments de tuple, chaque élément de tuple (rtx) de l'ensemble d'éléments de tuple (rtx) donnant une valeur caractéristique pour être captée par la cellule dédiée du capteur chimique et/ou dans une condition d'opération dédié du capteur chimique ou de la cellule du capteur chimique en présence de l'odeur et/ou du gaz représenté par le tuple de référence concerné (rt), et
- un identifiant (id) pour l'odeur et/ou le gaz représenté par le tuple de référence concerné (rt);
une unité d'évaluation (71, 3) pour comparer le tuple de mesures (mt) avec un ou plusieurs tuples de référence (rt) de l'ensemble de tuples de référence (rt) et faisant l'objet d'un résultat de la comparaison apportant un ou plusieurs identifiants d'odeur et/ou de gaz (id) au dispositif électronique portable,
le dispositif électronique portable étant un de:
un téléphone mobile (7),
un ordinateur portable,
un lecteur électronique,
un ordinateur tablette (1),
un contrôleur de jeu,
un dispositif de pointage,
un appareil photo ou vidéo,
une périphérique d'ordinateur,
le dispositif électronique portable contenant l'unité d'évaluation (71),
le dispositif électronique portable étant adapté à amener un ou plusieurs tuples de référence (rt) de la base de données (4) afin de le ou les comparer avec le tuple de mesures (mt), et
une liaison sans fil étant utilisée pour amener l'un ou les plusieurs tuples de référence (rt) de la base de données (4).

3. Système selon l'une des revendications précédentes,
comprenant un écran (6) pour montrer l'un ou les plusieurs identifiants d'odeur et/ou de gaz (id), et
particulièrement le dispositif électronique portable comprenant l'écran (6).

4. Système selon l'une des revendications précédentes,
le capteur chimique (12) comprenant un réseau de cellules de capteur (1122), et
particulièrement chaque cellule de capteur (1122) étant généralement sensible à un autre analyte que le capteur chimique (12).

5. Système selon l'une des revendications précédentes,
le dispositif électronique portable comprenant au moins un de
- un capteur de température pour compenser des variations de signal dépendantes de température dans un signal du capteur chimique (12), et
- un capteur d'humidité (1111) pour compenser des variations de signal dépendantes d'humidité dans un signal du capteur chimique (12).

6. Système selon l'une des revendications précédentes,
le dispositif électronique portable comprenant une unité d'entrée pour déclencher une mesure d'odeur et/ou de gaz.

7. Système selon l'une des revendications précédentes,
le capteur chimique (12) étant adapté à fournir des tuples de mesure (mt) de manière continue dans le temps.

8. Système selon l'une des revendications précédentes,
le dispositif électronique portable comprenant une unité de sélection pour sélectionner une ou plusieurs bases de données (76, 4) d'une pluralité de bases de données afin d'appliquer des tuples de référence (rt) d'elle ou d'elles.

9. Système selon l'une des revendications précédentes,
le capteur chimique (12) étant adapté à fournir le tuple de mesures (mt) en combinaison avec un identifiant du capteur chimique, et particulièrement en combinaison avec un horodatage.

10. Dispositif portable pour l'utilisation dans un système d'identification d'odeur et/ou de gaz selon l'une des revendications précédentes.

11. Procédé pour identifier une odeur et/ou un gaz, comprenant les étapes
de mesurer une odeur et/ou un gaz avec un dispositif électronique portable comprenant un capteur chimique (12), le capteur chimique (12) étant sensible à des différents analytes, le dispositif électronique portable étant un téléphone portable (7) ou un ordinateur portable ou un lecteur électronique ou un ordinateur tablette (1) ou un contrôleur de jeu ou un dispositif de pointage ou un appareil photo ou vidéo ou une périphérique d'ordinateur,
de fournir un tuple de mesures (mt) comprenant un ensemble d'éléments de tuple avec chaque élément de tuple (mtx) de l'ensemble d'éléments de tuple (mtx) donnant une valeur mesurée pendant une mesure d'odeur et/ou de gaz par une cellule dédié du capteur chimique et/ou dans une condition d'opération dédié du capteur chimique ou d'une cellule du capteur chimique;
le dispositif électronique portable envoyant le tuple de mesures (mt) par une liaison sans fil à une unité d'évaluation (3) arrangée loin du dispositif électronique portable,
l'unité d'évaluation (3) recevant le tuple de mesures (mt) du dispositif électronique et comparaissant le tuple de mesures (mt) avec un ou plusieurs tuples de référence (rt) d'une base de données, la base de données étant arrangée loin du dispositif électronique portable, chaque tuple de référence (rt) représentant une odeur et/ou un gaz et comprenant
- un ensemble d'éléments de tuple, chaque élément de tuple (rtx) de l'ensemble d'éléments de tuple (rtx) donnant une valeur caractéristique pour être captée par la cellule dédiée du capteur chimique et/ou dans une condition d'opération dédié du capteur chimique ou de la cellule du capteur chimique en présence de l'odeur et/ou du gaz représenté par le tuple de référence concerné (rt), et
- un identifiant (id) pour l'odeur et/ou le gaz représenté par le tuple de référence concerné (rt);
l'unité d'évaluation (3) renvoyant un ou plusieurs identifiants d'odeur et/ou du gaz par une liaison sans fil au dispositif électronique portable, faisant l'objet d'un résultat de la comparaison.

12. Procédé pour identifier une odeur et/ou un gaz, comprenant les étapes
de mesurer une odeur et/ou un gaz avec un dispositif électronique portable comprenant un capteur chimique (12), le capteur chimique (12) étant sensible à des différents analytes, le dispositif électronique portable étant un téléphone portable (7) ou un ordinateur portable ou un lecteur électronique ou un ordinateur tablette (1) ou un contrôleur de jeu ou un dispositif de pointage ou un appareil photo ou vidéo ou une périphérique d'ordinateur,
de fournir un tuple de mesures (mt) comprenant un ensemble d'éléments de tuple avec chaque élément de tuple (mtx) de l'ensemble d'éléments de tuple (mtx) donnant une valeur mesurée pendant une mesure d'odeur et/ou de gaz par une cellule dédié du capteur chimique et/ou dans une condition d'opération dédié du capteur chimique ou d'une cellule du capteur chimique;
une unité d'évaluation (71) incorporée dans le dispositif électronique portable amenant un ou plusieurs tuples de référence (rt) d'une base de données (4) par une liaison sans fil pour le ou les comparer avec le tuple de mesures (mt),
de comparer le tuple de mesure (mt) avec un ou plusieurs des tuples de référence (rt) amenés de la base de données, la base de données étant arrangée loin du dispositif électronique portable, chaque tuple de référence (rt) représentant une odeur et/ou un gaz et comprenant
- un ensemble d'éléments de tuple, chaque élément de tuple (rtx) de l'ensemble d'éléments de tuple (rtx) donnant une valeur caractéristique pour être captée par la cellule dédiée du capteur chimique et/ou dans une condition d'opération dédié du capteur chimique ou de la cellule du capteur chimique en présence de l'odeur et/ou du gaz représenté par le tuple de référence concerné (rt), et
- un identifiant (id) pour l'odeur et/ou le gaz représenté par le tuple de référence concerné (rt); et
retournant un ou plusieurs identifiants d'odeur et/ou da gaz au dispositif électronique portable faisant l'objet d'un résultat de la comparaison.

13. Procédé pour identifier une odeur et/ou un gaz selon la revendication 11 ou 12,
le tuple de mesures (mt) étant comparé avec l'un ou les plusieurs tuples de référence (rt) en déterminant une déviation entre des éléments du tuple de mesures (mtx) ensemble avec des éléments du tuple de référence (rtx) équivalents et un élément du tuple de mesures (mtx) ensemble avec son élément du tuple de référence (rtx) équivalent attribués à la même cellule du capteur chimique et/ou à la même condition d'opération du capteur chimique ou de la cellule du capteur chimique, et
l'identifiant (id) d'un tuple de référence (rt) comparé avec le tuple de mesures (mt) étant retourné faisant objet des déviations déterminées, et
particulièrement les éléments du tuple (mtx, rtx) à comparer comprenant des valeurs normalisées.

14. Procédé selon la revendication 13,
chaque déviation étant comparée avec un seuil (tx) attribué, et
l'identifiant (id) d'un tuple de référence (rt) comparé avec le tuple de mesures (mt) étant retourné en cas un numéro n de déviations est inférieur aux seuils attribués (tx).

15. Elément de programme d'ordinateur comprenant des moyens de code de programme d'ordinateur pour implémenter les étapes suivantes quand ils sont executés dans une unité de traitement d'un téléphone mobile (7) ou d'un ordinateur portable ou d'un lecteur électronique ou d'un ordinateur tablette (1) ou d'un contrôleur de jeu ou d'un dispositif de pointage ou d'un appareil photo ou vidéo ou d'une périphérique d'ordinateur:
recevoir un tuple de mesures (mt) d'une unité de capteur chimique (12) et comprenant un ensemble d'éléments de tuple avec chaque élément de tuple (mtx) de l'ensemble d'éléments de tuple donnant une valeur mesurée par une cellule dédié du capteur chimique et/ou dans une condition d'opération dédié du capteur chimique ou d'une cellule du capteur chimique pendant une mesure d'odeur et/ou de gaz;
amener un ou plusieurs tuples de référence (12) par une liaison sans fil d'une base de données, la base de données étant arrangée loin du dispositif électronique portable, chaque tuple de référence (rt) représentant une odeur et/ou un gaz et comprenant
- un ensemble d'éléments de tuple, chaque élément de tuple (rtx) de l'ensemble d'éléments de tuple (rtx) donnant une valeur caractéristique pour être captée par la cellule dédiée du capteur chimique et/ou dans une condition d'opération dédié du capteur chimique ou de la cellule du capteur chimique en présence de l'odeur et/ou du gaz représenté par le tuple de référence concerné (rt), et
- un identifiant (id) pour l'odeur et/ou le gaz représenté par le tuple de référence concerné (rt) comparant ledit tuple de mesures (mt) avec ledit un ou lesdits plusieurs tuples de référence (rt); et
retourner un ou plusieurs identifiants d'odeur et/ou de gaz (id) faisant objet d'un résultat de la comparaison.
